# FASCICULE DE BREVET EUROPEEN

(11) **EP 0 589 784 B1**
(45) Date de publication et mention de la délivrance du brevet: **17.12.1997**
(21) Numéro de dépôt: 93402310.2
(22) Date de dépôt: 21.09.1993
(51) Int. Cl.: C07D 333/54, C07D 333/58, A61K 31/38

(54) **Dérivé du 2-éthyl benzo [b] thiophène, son procédé de préparation et son utilisation comme intermédiaire de synthèse**
2-Ethylbenzo(b)thiphenederivat, Verfahren zu seiner Herstellung und seine Anwendung als Synthesezwischenprodukt
2-Ethyl-benzo(b)thiophene derivative, process of its preparation and its use as intermediate for synthesis

(30) Priorité: 22.09.1992 FR 9211276
(43) Date de publication de la demande: 30.03.1994
(73) Titulaire: SANOFI, 75008 Paris (FR)
(72) Inventeur: Dormoy, Jean-Robert, F-92330 Sceaux (FR)
(74) Mandataire: Polus, Camille

(56) Documents cités:
- EP-A- 0 279 263
- EP-A- 0 281 254
- EP-A- 0 345 592
- JOURNAL OF ORGANIC CHEMISTRY vol. 39, no. 19, 1974, COLUMBUS, OHIO pages 2828 -
- 2831 DONALD S. NOYCE ET AL. 'Reactivity of Benzo(b)thiophene in Electrophilic Reactions as Determined from solvolysis rates'

## Description

La présente invention se rapporte, d'une manière générale, à un nouveau dérivé du 2-éthyl-benzo[b]thiophène, à son procédé de préparation ainsi qu'à son utilisation comme intermédiaire de synthèse.

Plus précisément, l'invention a pour objet le 2-(1-bromo éthyl) benzo[b]thiophène de formule :

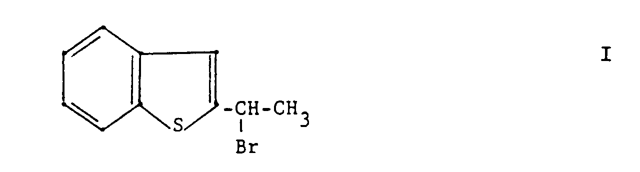

Le composé de l'invention s'est révélé particulièrement utile comme produit intermédiaire, notamment pour la préparation de 2-(1-amino éthyl) benzo[b]thiophènes N-substitués, notamment le 2-(1-benzyloxyamino éthyl) benzo[b]thiophène.

Ce dernier composé peut être lui-même utilisé comme intermédiaire dans la préparation de différents produits notamment pour la synthèse finale de la N-hydroxy N-{1-(benzo[b]thièn-2 yl) éthyl}urée de formule :

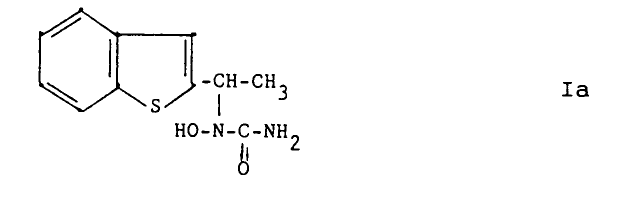

Ce dérivé d'urée, qui a été décrit dans la demande de brevet EP-0 279 263, s'est révélé particulièrement intéressant pour ses propriétés pharmacologiques, notamment ses propriétés inhibitrices des leukotriènes pouvant le rendre utile dans le domaine de l'asthme, de la polyarthrite rhumatoïde et des maladies inflammatoires du colon.

On a décrit dans la demande de brevet EP-0 279 263 citée précédemment, plusieurs voies de synthèse du composé de formule Ia.

L'une d'entre elles fait appel à la suite de réactions suivantes :
a) chauffage du 2-(1-chloro éthyl) benzo[b]thiophène avec la O-benzyl-hydroxylamine, dans un solvant tel que le diméthylsulfoxyde ou le tétrahydrofuranne, pour donner le 2-(1-benzyloxyamino éthyl) benzo[b]thiophène de formule : 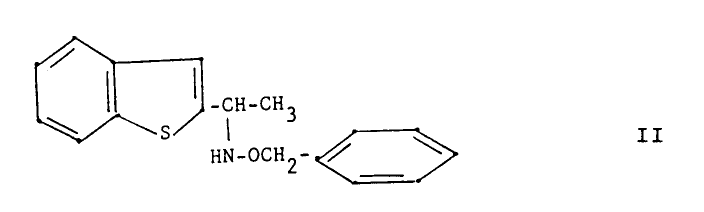
b) réaction du dérivé benzyloxy ainsi formé avec le triméthylsilylisocyanate pour former la N-benzyloxy N-{1-(benzo[b]thièn-2 yl) éthyl}urée ;
c) hydrogénation catalytique sur charbon palladié, de ce dérivé d'urée, pour obtenir finalement le composé de formule Ia.

Cependant, aucun exemple ne décrit la mise en oeuvre de ce procédé pour pouvoir juger de sa faisabilité sur le plan industriel.

En outre, aucune méthode n'a été rapportée dans cette demande de brevet pour la préparation du composé de départ, à savoir le 2-(1-chloro éthyl) benzo[b]thiophène.

Ce dérivé chloré est toutefois un composé connu, ayant été décrit dans J. Org. Chem. 1974 39 (18) pp. 2828-2831, de même qu'un procédé pour sa préparation.

Selon ce procédé, le 2-(1-chloro éthyl) benzo[b]thiophène est obtenu avec un rendement de 80% après traitement du 2-(1-hydroxy éthyl) benzo[b]thiophène au moyen de chlorure de thionyle, cet alcool étant lui-même préparé avec un rendement de 44% à partir du benzo[b]thiophène. En conséquence, le 2-(1-chloro éthyl) benzo[b]thiophène a pu être obtenu par mise en oeuvre de ce procédé selon un rendement global de 35% maximum.

Dans le cadre de l'élaboration de l'invention, on a tenté de préparer le 2-(1-benzyloxyamino éthyl) benzo[b]thiophène par réaction du 2-(1-chloro éthyl) benzo[b]thiophène et de l'O-benzylhydroxylamine selon le schéma réactionnel de la demande de brevet européen cité précédemment.

Ce produit a pu être synthétisé avec toutefois des rendements ne dépassant pas 57%.

La recherche d'un procédé industriel pour la préparation du 2-(1-benzyloxyamino éthyl) benzo[b]thiophène, mettant en oeuvre des intermédiaires de synthèse d'accés facile et peu onéreux ainsi qu'un rendement satisfaisant en produit final reste d'un intérêt incontestable.

Or, on a trouvé de manière tout à fait surprenante, selon l'invention, que le 2-(1-bromo éthyl) benzo[b]thiophène peut donner accès au composé de formule II avec facilité et avec de très bons rendements, les rendements obtenus étant de l'ordre de 75%.

De plus, on a trouvé que le 2-(1-bromo éthyl) benzo[b]thiophène peut être lui-même obtenu de manière plus avantageuse que le dérivé 1-chloro correspondant, et avec des rendements supérieurs à ce dernier.

Selon l'invention, on prépare le 2-(1-bromo éthyl) benzo[b]thiophène en faisant réagir, dans un solvant approprié, le 2-éthyl benzo[b]thiophène avec un agent bromant en présence d'un initiateur de radicaux libres.

Généralement, on utilise comme agent bromant le N-bromosuccinimide ou la 1,3-dibromo 5,5-diméthyl hydantoïne, et comme initiateur de radicaux libres l'azobisisobutyronitrile.

En outre, la réaction a lieu de préférence à une température comprise entre la température ambiante et la température de reflux du milieu.

Comme solvant, on utilise en général un solvant apolaire tel que par exemple le tétrachlorure de carbone, le cyclohexane ou l'heptane.

Quant au 2-éthyl benzo[b]thiophène de départ, il s'agit d'un produit connu, largement décrit dans la littérature chimique.

Comme indiqué précédemment, on a trouvé que le 2-(1-bromo éthyl) benzo[b]thiophène de l'invention présente plusieurs avantages sur son homologue chloré, tant au niveau de sa préparation que de sa mise en oeuvre comme intermédiaire de synthèse pour la préparation du dérivé benzyloxy de formule II.

Ces avantages ont pu être déduits de différents essais comparatifs effectués dans le cadre de l'élaboration de la présente invention.

Par exemple, on a préparé le 2-(1-bromo éthyl) benzo[b]thiophène et son homologue 1-chloro selon un procédé strictement analogue consistant à faire réagir 1 milliéquivalent molaire de 2-éthyl benzo[b]thiophène dans 13 volumes d'heptane, avec 0,55 milliéquivalent molaire d'agent halogénant, c'est-à-dire de 1,3-dibromo 5,5-diméthyl hydantoïne ou de 1,3-dichloro 5,5-diméthyl hydantoïne, en présence de X% d'azobisisobutyronitrile (AIBN), à une température T et durant Y heures.

Cette réaction, schématisée ci-dessous, a fourni un mélange de différents produits, à savoir les composés A à D ci-dessous : 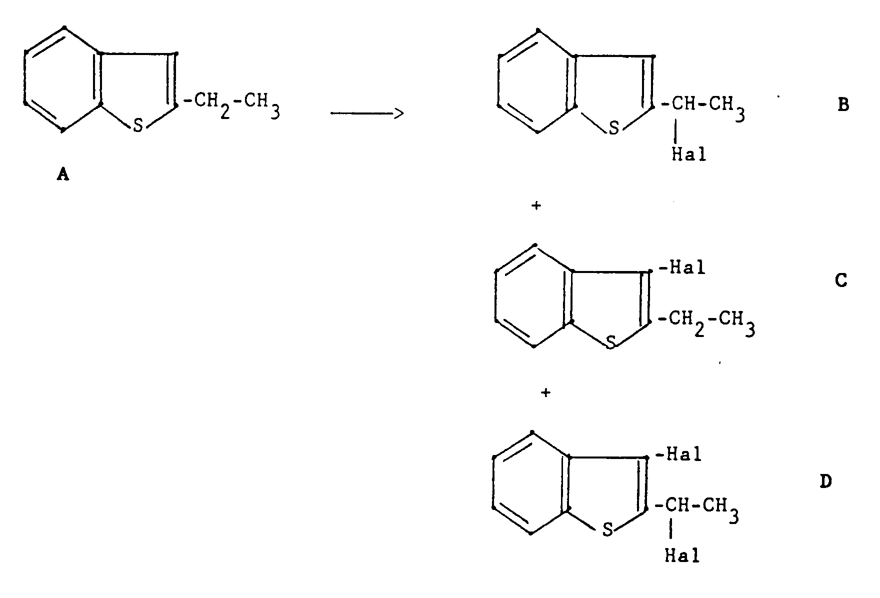 Hal représentant un atome de chlore ou de brome.

On a obtenu les résultats suivants :

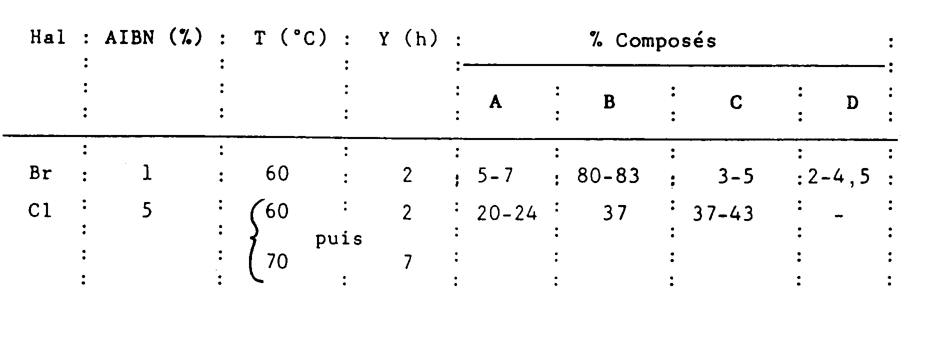

Ces résultats montrent que la sélectivité de la conversion du 2-éthyl benzo[b]thiophène en dérivé α-halogéné est supérieure dans le cas de la bromation. Cette sélectivité se traduit par des rendements supérieurs à 80% en 2-(1-bromo éthyl) benzo[b]thiophène, comparativement à des rendements inférieurs à 40% en 2-(1-chloro éthyl) benzo[b]thiophène.

En outre, l'utilisation d'une quantité supérieure d'AIBN se révèle nécessaire pour la chloration.

Comme indiqué précédemment, le 2-(1-bromo éthyl) benzo[b]thiophène peut donner accès au dérivé benzyloxy de formule II.

En conséquence, un autre objet de l'invention se rapporte à la préparation du 2-(1 benzyloxyamino éthyl) benzo[b]thiophène par mise en oeuvre d'un procédé selon lequel on fait réagir le 2-(1-bromo éthyl) benzo[b]thiophène avec l'O-benzylhydroxylamine.

Généralement, cette réaction se déroule à la température ambiante et dans un solvant polaire tel que le tétrahydrofuranne, le N,N-diméthylformamide, le diméthylsulfoxyde ou l'acétonitrile.

Des essais comparatifs ont permis de mettre en évidence la supériorité du dérivé 1-bromo de formule I sur son homologue 1-chloro dans la préparation du 2-(1-benzyloxyamino éthyl) benzo[b]thiophène.

A cet effet, on a fait réagir 1 équivalent en mole de 2-(1-bromo éthyl) benzo[b]thiophène ou de 2-(1-chloro éthyl) benzo[b]thiophène avec Z équivalents en mole d'O-benzylhydroxylamine dans le tétrahydrofuranne, à une température T₁ et durant Y₁ heures.

Cette réaction, schématisée ci-dessous, a permis d'obtenir le composé E ou composé désiré : 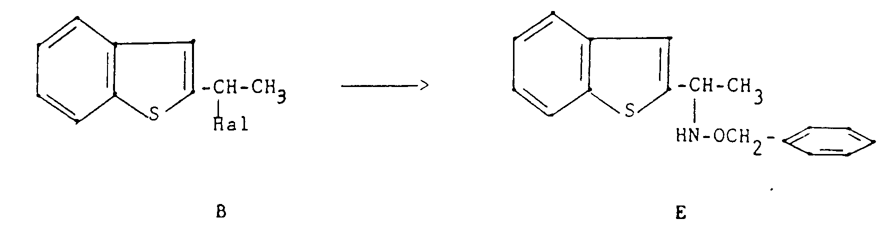 Hal désignant un atome de chlore ou de brome.

On a enregistré les résultats suivants :

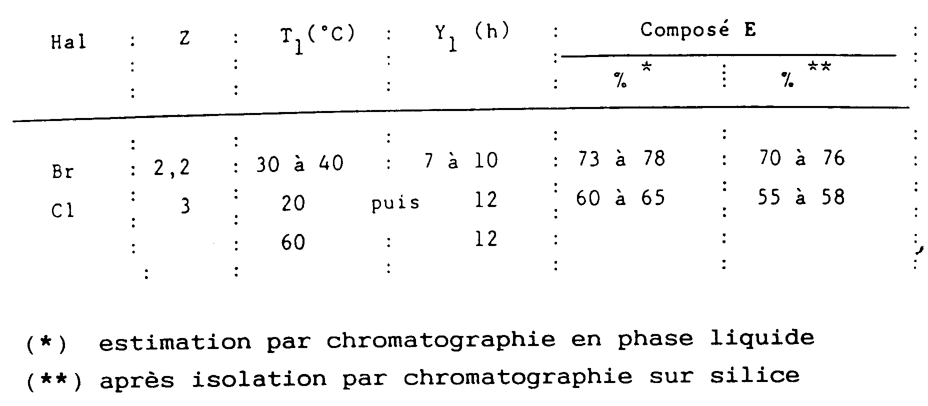

Ces résultats montrent que la mise en oeuvre du composé chloré B pour accéder au composé E désiré nécessite des conditions de réaction plus dures se traduisant par un chauffage prolongé à 60°C au lieu de 30 à 40°C dans le cas du composé bromé B correspondant.

En outre, comparativement au composé B bromé, le composé B chloré conduit à un rendement moindre en composé E, avec augmentation du taux de produits secondaires.

Comme mentionné précédemment, le 2-(1-bromo éthyl) benzo[b]thiophène peut être utilisé également pour la préparation de la N-hydroxy N-{1-benzo[b]thièn-2 yl)éthyl}urée de formule Ia.

En conséquence, l'invention se rapporte au 2-(1-bromo éthyl) benzo[b]thiophène en tant qu'intermédiaire pour la synthèse finale du dérivé d'urée de formule Ia.

Par exemple, on peut préparer ce dérivé d'urée de formule Ia au départ du dérivé benzyloxy de formule II, obtenu lui-même selon l'invention à partir du 2-(1-bromo éthyl) benzo[b]thiophène, par mise en oeuvre d'un procédé comportant les étapes suivantes :
a) on traite le dérivé benzyloxy de formule II d'abord avec le triméthylsilylisocyanate dans un solvant approprié tel qu'un solvant polaire, par exemple un éther, et à la température de reflux du milieu ensuite avec une solution aqueuse de chlorure d'ammonium pour obtenir la N-benzyloxy N-{1-benzo[b]thièn-2 yl) éthyl}urée ;
b) on hydrogène ce dérivé d'urée en présence d'un catalyseur tel que le charbon palladié, éventuellement en présence de formiate d'ammonium, dans un solvant approprié tel que l'acide acétique, et à une température de 20 à 50°C, pour obtenir finalement le composé désiré de formule Ia.

Les Exemples non limitatifs suivants illustrent l'invention :

### Exemple 1

### Préparation du 2-(1-bromo éthyl) benzo[b]thiophène :

A un mélange de 9,43 g (33 mmoles) de 1,3-dibromo 5,5-diméthyl hydantoïne, 0,49 g (3 mmoles) d'azobisisobutyronitrile dans 117,5 ml d'heptane préchauffé à 60°C, on ajoute, en 10 minutes, une solution de 9,78 g (60 mmoles) de 2-éthyl benzo[b]thiophène dans 10 ml d'heptane. On maintient le mélange durant 1 heure supplémentaire à cette température. Après refroidissement à 20°C, on poursuit l'agitation pendant 1 heure, puis on essore et lave avec 10 ml d'heptane la 5,5-diméthyl hydantoïne qui a précipité. On lave le filtrat avec une solution aqueuse de thiosulfate de sodium (3 g/100 ml), puis avec 3 fois 100 ml d'eau. Après séchage sur sulfate de sodium, on concentre la phase heptanique jusqu'à environ 40 ml, puis on refroidit à -10°C pendant une heure et demie. On essore le précipité formé et on le sèche sous vide partiel à 20°C pour fournir 9,05 g du composé souhaité sous la forme d'une poudre beige, ce qui représente un rendement de 57% (P.F. : 53°C, titre par chromatographie en phase liquide : 92%). Un second jet moins pur est obtenu par concentration des eaux-mères (16%).

De cette manière, on obtient le 2-(1-bromo éthyl) benzo[b]thiophène avec un rendement de 73%.
Spectre R.M.N.¹H (CDCl₃, 300 MHz) :
2,18 (d,J = 6,7 Hz, 3H, CH₃)
5,57 (q, J = 6,7 Hz, 1H, CHBr)
7,25 - 7,45 (m, 3H aromatiques)
7,65 - 7,85 (m, 2H aromatiques)
Spectre R.M.N.¹³C (CDCl₃, 75 MHz) :
27,65 (CH₃) ; 43,69 (CHBr) ; 121, 74 ; 122, 54 ; 123, 99 ; 124, 68 ; 125, 02 (5 CH aromatiques) ; 139, 01 ; 139, 73 ; 148, 02 (3C aromatiques quaternaires).

### Exemple 2

### Préparation de la N-hydroxy N-{1-(benzo[b]thièn-2 yl) éthyl}urée

### a) 2-(1-benzyloxyamino éthyl) benzo[b]thiophène :

Sous atmosphère d'azote, on met en solution 1 g (4 mmoles) de 2-(1-bromo éthyl) benzo[b]thiophène (titre : 97%) et 1,48 g (12 mmoles) d'O-benzyl-hydroxylamine dans 2 ml de tétrahydrofuranne anhydre. On agite le mélange pendant 5,5 heures à 20°C. Après ajout de 15 ml d'eau, on acidifie le milieu réactionnel à pH = 3 avec de l'acide chlorhydrique dilué, et on extrait avec 4 fois 15 ml d'éther diéthylique. On sèche la phase éthérée sur sulfate de sodium et on concentre sous pression réduite, ce qui donne 1,44 g d'une huile brune. On filtre ensuite sur colonne de silice (éluant gradient de dichlorométhane dans l'hexane 1/99 à 30/70).

De cette manière, on obtient 0,89 g de 2-(1-benzyloxyamino éthyl) benzo[b]thiophène sous la forme d'un solide beige rosé.
P.F. : 55-56°C
Rendement : 76%
Spectre R.M.N. ¹H (CDCl₃, 300 MHz) :
1,59 (d, J = 6,6 Hz, 3H, CH₃ CH)
4,58 (q, J = 6,6 Hz, 1H CH CH₃)
4,83 (s, 2H, CH₂, phényle)
5,75 (large s, 1H, NH)
7,26 - 7,50 (m, 8H aromatiques)
7,78 - 7,92 (m, 2H aromatiques)
Spectre R.M.N.¹³C (CDCl₃, 75 MHz) :
20, 31 (CH₃) ; 56,78 (CHN) ; 76,87
(CH₂-phényle) ; 120,89 ; 122,36 ; 123,25 ; 123,94 ; 124,08 ; 127,80 (6 CH aromatiques) ;
128,32 et 128,52 (2C ortho + 2C méta de CH₂phényle) ; 137,69 ; 139,36 ; 139,51 ; 147,77 (4C aromatiques quaternaires).

### b) N-benzyloxy N-{1-(benzo[b]thièn-2 yl) éthyl}urée

Dans 25 ml de tétrahydrofuranne, on dissout 10,1 g (33,6 mmoles) de 2-(1-benzyloxyamino éthyl) benzo[b]thiophène (titre environ 94%) et 12,5 g (3,3 équ. mol.) de triméthylsilylisocyanate. On chauffe le mélange à reflux pendant 48 heures.

Après refroidissement à 25°C, on ajoute 150 ml d'une solution aqueuse saturée de chlorure d'ammonium, on poursuit l'agitation pendant 1 heure et on extrait le mélange avec deux fois 50 ml d'acétate d'éthyle. On rassemble les phases organiques et on lave avec deux fois 25 ml d'eau. On sèche sur sulfate de sodium, on évapore à sec et on purifie le résidu par chromatographie sur silice.

De cette manière, on obtient 5,9 g de N-benzyloxy N{1-(benzo[b]thièn-2 yl) éthyl}urée sous forme d'une poudre crème.
P.F. : 126°C
Rendement : 54%
Spectre I.R. (KBr) : 3420 (NH₂), 1661 (C=O)
Spectre R.M.N.¹H (300 MHz, DMSOD₆) :
1,6 (d, J = 7Hz, 3H, CH₃)
4,65 - 4,85 (m, 2H, OCH₂)
5,55 (q, J = 7Hz, 1H, CH CH₃)
6,7 (large s, 2H, NH₂)
7,2 - 7,5 (m, 8H aromatiques)
7,70 - 7,95 (2m, 2H aromatiques)
Spectre R.M.N.¹³C (75 MHz, DMSOD₆) :
17,68 (CH₃) ; 53,91 (CHN) ; 78,03 (OCH₂) ; 121,95 ; 122,25 ; 123,41 ; 124,21 ; 124,25 ; 128,22 ; 128,30 (x2) ; 129,15 (x2) ; (10 CH aromatiques) 135,47 ; 138,92 ; 138,96 ; 145,28 (4C aromatiques quaternaires).

### c) N-hydroxy N-{1-(benzo[b]thièn-2 yl) éthyl}urée

Dans un réacteur, on introduit 2,08 g (6 mmoles) de N-benzyloxy N-{1-(benzo[b]thièn-2 yl) éthyl}urée (titre environ 94%), 1 g de charbon palladié à 10%, 62,5 ml d'acide acétique glacial et 6 gouttes d'acide chlorhydrique 12N.

On maintient le mélange pendant 24 heures à 50°C sous une pression d'hydrogène de 5 bars. Après refroidissement, essorage du catalyseur sur terres de diatomées, neutralisation avec de l'hydroxyde de sodium, extraction avec de l'acétate d'éthyle et évaporation du solvant, on purifie le résidu par chromatographie sur silice (éluant : gradient d'acétate d'éthyle dans l'hexane 50/50 à 100/0).

De cette manière, on obtient 0,16 g de N-hydroxy N-{1-(benzo[b]thièn-2 yl) éthyl}urée sous forme d'un solide beige.
P.F. : 154°C
Rendement : 11%
Spectre I.R. (KBr) : 3475 (NH₂), 1666 (C=O)
Spectre R.M.N.¹H (300 MHz, DMSOD₆) :
1,51 (d, J = 7,1 Hz, 3H, CH₃) ; 5,56 (q, J = 7,1 Hz, 1H CH-CH₃) ; 6,45 (larges s, 2H, NH₂) 7,25-7,45 (m, 3H aromatiques) ; 7,70-7,95 (m, 2H aromatiques) ; 9,27 (s, 1H, OH)
Spectre de masse (CI-CH₄) :
237 (MH)⁺, 251 (MCH₃)⁺, 265 (MC₂H₅)⁺

## Revendications

1. 2-(1-bromo éthyl) benzo[b]thiophène de formule :

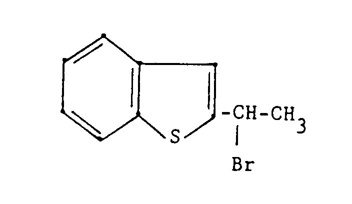

2. Procédé de préparation du 2-(1-bromo éthyl) benzo[b]thiophène, caractérisé en ce que l'on fait réagir, dans un solvant, le 2-éthyl benzo[b]thiophène avec un agent bromant, en présence d'un initiateur de radicaux libres, ce qui fournit le composé désiré.

3. Procédé selon la revendication 2, caractérisé en ce que le solvant est un solvant apolaire.

4. Procédé selon la revendication 3, caractérisé en ce que le solvant apolaire est le tétrachlorure de carbone, le cyclohexane ou l'heptane.

5. Procédé selon l'une des revendications 2 à 4, caractérisé en ce que l'initiateur de radicaux libres est l'azobisisobutyronitrile.

6. Procédé selon l'une des revendications 2 à 5, caractérisé en ce que l'agent bromant est le N-bromosuccinimide ou la 1,3-dibromo 5,5-diméthyl hydantoïne.

7. Procédé selon l'une des revendications 2 à 6, caractérisé en ce que la réaction a lieu à une température comprise entre la température ambiante et la température de reflux du milieu.

8. Procédé de préparation du 2-(1-benzyloxyamino éthyl) benzo[b]thiophène, caractérisé en ce que l'on fait réagir à température ambiante et dans un solvant, le 2-(1-bromo éthyl) benzo[b]thiophène avec l'O-benzylhydroxylamine, ce qui fournit le composé désiré.

9. Procédé selon la revendication 8, caractérisé en ce que le solvant est un solvant polaire.

10. Procédé selon la revendication 9, caractérisé en ce que le solvant polaire est le tétrahydrofuranne, le N,N-diméthylformamide, le diméthylsulfoxyde ou l'acétonitrile.

11. Procédé de préparation de la N-hydroxy N-{1-(benzo[b]thièn-2 yl) éthyl}urée, caractérisé en ce que :
a) on fait réagir, à température ambiante et dans un solvant, le 2-(1-bromo éthyl) benzo[b]thiophène avec l'O-benzylhydroxylamine, pour obtenir le 2-(1-benzyloxyamino éthyl) benzo[b]thiophène ;
b) on traite le dérivé benzyloxyamino ainsi obtenu, d'abord avec le triméthylsilylisocyanate dans un solvant et à la température de reflux du milieu, ensuite avec une solution aqueuse de chlorure d'ammonium, ce qui fournit la N-benzyloxy N-{1-(benzo[b]thièn-2 yl) éthyl}urée ;
c) on hydrogène le dérivé d'urée ainsi obtenu en présence d'un catalyseur dans un solvant et à une température de 20 à 50°C, ce qui fournit le composé désiré.

12. Procédé selon la revendication 11, caractérisé en ce que le catalyseur est le charbon palladié, éventuellement en présence de formiate d'ammonium.

## Claims

1. 2-(1-bromo-ethyl)-benzo[b]thiophene of formula:

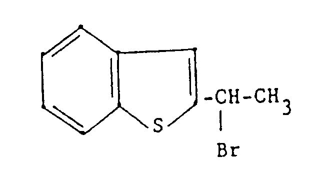

2. Process for preparing 2-(1-bromo-ethyl)-benzo[b]thiophene, characterised in that 2-ethyl-benzo[b]thiophene is reacted, in a solvent, with a brominating agent, in the presence of a free radical initiator, to yield the desired compound.

3. Process according to claim 2, characterised in that the solvent is an apolar solvent.

4. Process according to claim 3, characterised in that the apolar solvent is carbon tetrachloride, cyclohexane or heptane.

5. Process according to one of claims 2 to 4, characterised in that the free radical initiator is azobisisobutyronitrile.

6. Process according to one of claims 2 to 5, characterised in that the brominating agent is N-bromosuccinimide or 1,3-dibromo-5,5-dimethyl-hydantoine.

7. Process according to one of claims 2 to 6, characterised in that the reaction takes place at a temperature between ambient temperature and the reflux temperature of the medium.

8. Process for preparing 2-(1-benzyloxyamino-ethyl)-benzo[b]thiophene, characterised in that 2-(1-bromo-ethyl)-benzo[b]thiophene is reacted with O-benzylhydroxylamine at ambient temperature and in a solvent, to yield the desired compound.

9. Process according to claim 8, characterised in that the solvent is a polar solvent.

10. Process according to claim 9, characterised in that the polar solvent is tetrahydrofuran, N,N-dimethylformamide, dimethylsulphoxide or acetonitrile.

11. Process for preparing N-hydroxy-N-{1-(benzo[b]thien-2-yl)-ethyl}urea, characterised in that:
a) 2-(1-bromo-ethyl)-benzo[b]thiophene is reacted with O-benzylhydroxylamine at ambient temperature and in a solvent, to obtain 2-(1-benzyloxyamino-ethyl)-benzo[b]thiophene;
b) the benzyloxyamino derivative thus obtained is treated first with trimethylsilylisocyanate in a solvent and at the reflux temperature of the medium, then with an aqueous ammonium chloride solution, to yield N-benzyloxy-N-{1-(benzo[b]thien-2-yl)ethyl}urea;
c) the urea derivative thus obtained is hydrogenated in the presence of a catalyst in a solvent and at a temperature of 20 to 50°C, to yield the desired compound.

12. Process according to claim 11, characterised in that the catalyst is palladium/charcoal, optionally in the presence of ammonium formate.

## Patentansprüche

1. 2-(1-Bromethyl)-benzo[b]thiophen der Formel:

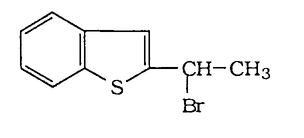

2. Verfahren zur Herstellung von 2-(1-Bromethyl)-benzo[b]thiophen, dadurch gekennzeichnet, daß man 2-Ethyl-benzo[b]thiophen in einem Lösungsmittel in Gegenwart eines freie Radikale liefernden Initiators mit einem Bromierungsmittel unter Bildung der gewünschten Verbindung umsetzt.

3. Verfahren nach Anspruch 2, dadurch gekennzeichnet, daß das Lösungsmittel ein apolares Lösungsmittel ist.

4. Verfahren nach Anspruch 3, dadurch gekennzeichnet, daß das apolare Lösungsmittel Tetrachlorkohlenstoff, Cyclohexan oder Heptan ist.

5. Verfahren nach einem der Ansprüche 2 bis 4, dadurch gekennzeichnet, daß der freie Radikale liefernde Initiator Azobisisobutyronitril ist.

6. Verfahren nach einem der Ansprüche 2 bis 5, dadurch gekennzeichnet, daß das Bromierungsmittel N-Bromsuccinimid oder 1,3-Dibrom-5,5-dimethylhydantoin ist.

7. Verfahren nach einem der Ansprüche 2 bis 6, dadurch gekennzeichnet daß die Reaktion bei einer Temperatur zwischen der Umgebungstemperatur und der Rückflußtemperatur des Mediums durchgeführt wird.

8. Verfahren zur Herstellung von 2-(1-Benzyloxyaminoethyl)-benzo[b]thiophen, dadurch gekennzeichnet, daß man 2-(1-Bromethyl)-benzo[b]thiophen bei Umgebungstemperatur und in einen, Lösungsmittel unter Bildung der gewünschten Verbindung mit O-Benzylhydroxylamin umsetzt.

9. Verfahren nach Anspruch 8, dadurch gekennzeichnet, daß das Lösungsmittel ein polares Lösungsmittel ist.

10. Verfahren nach Anspruch 9, dadurch gekennzeichnet, daß das polare Lösungsmittel Tetrahydrofuran, N,N-Dimethylformamid, Dimethylsulfoxid oder Acetonitril ist.

11. Verfahren zur Herstellung von N-Hydroxy-N-{1-(benzo[b]thien-2-yl)-ethyl}-harnstoff, dadurch gekennzeichnet, daß man:
a) 2-(1-Bromethyl)-benzo[b]thiophen bei Umgebungstemperatur und in einem Lösungsmittel mit O-Benzylhydroxylamin umsetzt zur Bildung von 2-(1-Benzyloxyaminoethyl)-benzo[b]thiophen;
b) das in dieser Weise erhaltene Benzyloxyamino-Derivat zunächst mit Trimethylsilylisocyanat in einem Lösungsmittel und bei der Rückflußtemperatur des Lösungsmittels umsetzt und anschließend mit einer wäßrigen Lösung von Ammoniumchlorid zur Bildung von N-Benzyloxy-N-{1-(benzo[b]thien-2-yl)-ethyl}-harnstoff:
c) man das in dieser Weise erhaltene Harnstoffderivat in Gegenwart eines Katalysators in einem Lösungsmittel und bei einer Temperatur von 20 bis 50°C hydriert zur Bildung der gewünschten Verbindung.

12. Verfahren nach Anspruch 11, dadurch gekennzeichnet, daß der Katalysator Palladium-auf-Kohlenstoff, gegebenenfalls in Gegenwart von Ammoniumformiat, ist.
